Europäisches Patentamt
European Patent Office
Office européen des brevets

(19) (11) Veröffentlichungsnummer: **0 048 850**
**A1**

(12) **EUROPÄISCHE PATENTANMELDUNG**

(21) Anmeldenummer: **81107095.2**

(22) Anmeldetag: **09.09.81**

(51) Int. Cl.³: **C 07 D 249/18**, C 07 D 249/22, C 07 D 249/16, C 07 D 401/12, C 07 D 403/12, C 07 D 413/12, A 01 N 43/64, A 01 N 43/72, C 07 D 453/06

(30) Priorität: **19.09.80 DE 3035391**
**13.06.81 DE 3123534**

(43) Veröffentlichungstag der Anmeldung: **07.04.82**
**Patentblatt 82/14**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Förster, Heinz, Dr., Am Eckbusch 47, D-5600 Wuppertal 1 (DE)**
Erfinder: **Hofer, Wolfgang, Dr., Pahlkestrasse 59, D-5600 Wuppertal 1 (DE)**
Erfinder: **Maurer, Fritz, Dr., Roeberstrasse 8, D-5600 Wuppertal 1 (DE)**
Erfinder: **Mues, Volker, Dr., Gellertweg 13, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert, Dr., Im Waldwinkel 110, D-5060 Berg.-Gladbach 2 (DE)**

(54) **1-Benztriazolyloxy-essigsäureamide, Verfahren zu ihrer Herstellung und ihre Verwendung als Herbizide.**

(57) Neue 1-Benztriazolyloxy-essigsäureamide der allgemeinen Formel I

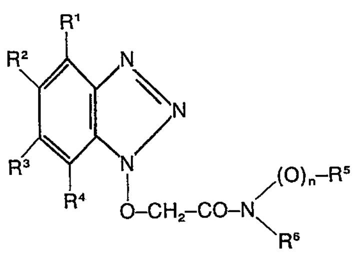

(I)

worin

$R^1$, $R^2$, $R^3$ und $R^4$, welche gleich oder verschieden sein können, für Wasserstoff, Cyano, Nitro, Halogen, Amino oder gegebenenfalls halogensubstituiertes (Di)Alkylamino, Carbamoyl, Monoalkylamino- oder Dialkylamino-carbonyl, Aminosulfonyl, Monoalkylamino- oder Dialkylaminosulfonyl, für gegebenenfalls durch Halogen, Cyano, Alkoxy oder Alkylthio substituiertes Alkyl, oder für gegebenenfalls halogen-substituiertes Alkoxy, Alkylthio oder Alkylsulfonyl stehen, oder in welcher zwei benachbarte Reste $R^1$ und $R^2$, $R^2$ und $R^3$ oder $R^3$ und $R^4$ zusammen für Alkylen oder Benzo stehen,

n für Null oder 1 steht und

$R^5$ und $R^6$, welche gleich oder verschieden sein können, einzeln für gegebenenfalls substituierte Reste aus der Reihe Alkyl, Alkenyl, Alkinyl, Cycloalk(en)yl, Aralkyl oder Aryl stehen oder – für den Fall, dass n für Null steht – zusammen mit dem Stickstoffatom, an das sie gebunden sind, einen gegebenenfalls substituierten, gegebenenfalls teilweise ungesättigten und gegebenenfalls benzannelierten Mono- oder Bicyclus bilden, der gegebenenfalls weitere Heteroatome enthält,

und ein Verfahren zu ihrer Herstellung, ausgehend von entsprechenden 1-Hydroxy-benztriazolen und Halogen-essigsäureamiden.

Die neuen Verbindungen der Formel (I) können insbesondere als Herbizide verwendet werden.

EP 0 048 850 A1

BAYER AKTIENGESELLSCHAFT 5090 Leverkusen, Bayerwerk

Zentralbereich
Patente, Marken und Lizenzen Bi-by
Ia

# 1-Benztriazolyloxy-essigsäureamide, Verfahren zu ihrer Herstellung und ihre Verwendung als Herbizide

Die Erfindung betrifft neue 1-Benztriazolyloxy-essigsäureamide, ein Verfahren zu ihrer Herstellung und ihre Verwendung als Herbizide.

Es ist bereits bekannt geworden, daß bestimmte Phenoxycarbonsäureamide, wie z.B. 2,4-Dichlorphenoxy-essigsäureamid, herbizid wirksam sind (vgl. FR-PS 1 313 84o). Die als Herbizide bekannten Phenoxycarbonsäureamide zeigen jedoch bei den üblichen Aufwandmengen nur eine geringe Wirkung gegen Ungräser und können zur Bekämpfung von Unkräutern in verschiedenen Kulturen wegen mangelnder Selektivität nicht verwendet werden.

Es wurden nun neue 1-Benztriazolyloxy-essigsäureamide der Formel I

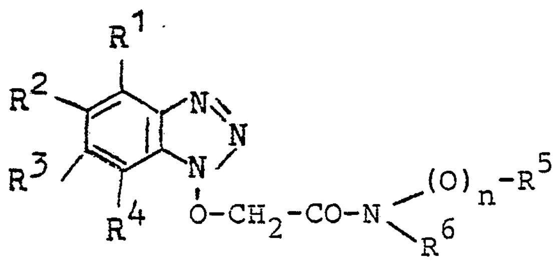

(I)

gefunden, in welcher

$R^1$, $R^2$, $R^3$ und $R^4$, welche gleich oder verschieden sein können, für Wasserstoff, Cyano, Nitro, Halogen, Amino oder gegebenenfalls halogensubstituiertes (Di)Alkylamino, Carbamoyl, Monoalkylamino - oder Dialkylamino-carbonyl, Aminosulfonyl, Monoalkylamino- oder Dialkylaminosulfonyl, für gegebenenfalls durch Halogen, Cyano, Alkoxy oder Alkylthio substituiertes Alkyl, oder für gegebenenfalls halogen-substituiertes Alkoxy, Alkylthio oder Alkylsulfonyl stehen, oder in welcher zwei benachbarte Reste $R^1$ und $R^2$, $R^2$ und $R^3$ oder $R^3$ und $R^4$ zusammen für Alkylen oder Benzo stehen,

n für Null oder 1 steht und

$R^5$ und $R^6$, welche gleich oder verschieden sein können, einzeln für gegebenenfalls substituierte Reste aus der Reihe Alkyl, Alkenyl, Alkinyl, Cycloalk(en)yl, Aralkyl oder Aryl stehen oder - für den Fall, daß n für Null steht - zusammen mit dem Stickstoffatom, an das sie gebunden sind, einen gegebenenfalls substituierten, gegebenenfalls teilweise ungesättigten und gegebenenfalls benzannelierten Mono- oder Bicyclus bilden, der gegebenenfalls weitere Heteroatome enthält.

Man erhält die neuen 1-Benztriazolyloxy-essigsäureamide der Formel (I), wenn man Hydroxy-benztriazole der Formel II

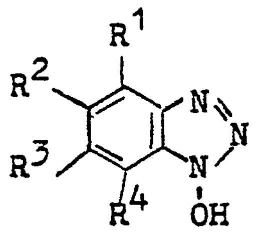

(II)

in welcher

$R^1$, $R^2$, $R^3$ und $R^4$ die oben angegebenen Bedeutungen haben,

mit Halogen-essigsäureamiden der Formel III

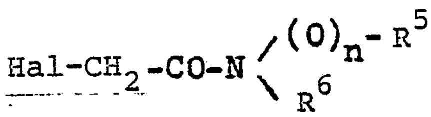

(III)

in welcher

n, $R^5$ und $R^6$ die oben angegebene Bedeutungen haben und

Hal für Chlor oder Brom steht,

gegebenenfalls in Gegenwart eines Säureakzeptors und gegebenenfalls unter Verwendung eines Verdünnungsmittels umsetzt.

Die neuen 1-Benztriazolyloxy- essigsäureamide der Formel (I) zeichnen sich durch starke herbizide Wirksamkeit aus; zum Teil sind sie auch zur Regulierung des Wachstums von Nutzpflanzen und zur Bekämpfung pilzlicher Pflanzenkrankheiten, wie z.B. pyricularia oryzaein Reis, geeignet.

Überraschenderweise zeigen die neuen 1-Benztriazolyloxy-essigsäureamide eine wesentlich bessere und andersartige herbizide Wirkung als die aus dem Stand der Technik bekannten Phenoxycarbonsäureamide. Insbesondere überrascht die Tatsache, daß die erfindungsgemäßen Verbindungen bei guter Verträglichkeit gegenüber Nutzpflanzen neben ihrer hohen Wirkung gegen dikotyle Unkräuter auch sehr gute Wirkung gegen Ungräser - einschließlich Cyperus - zeigen, während konstitutionell ähnliche Phenoxy-alkancarbonsäurederivate, wie z.B. 2,4-Dichlorphenoxy-essigsäureamid, nur geringe Wirkung gegen Gramineen aufweisen. Die neuen Wirkstoffe eignen sich außerdem zur selektiven Unkrautbekämpfung in Rüben, Baumwolle, Mais, Reis, Weizen und anderen Getreidearten.

Gegenstand der vorliegenden Erfindung sind vorzugsweise 1-Benztriazolyloxy-essigsäureamide der Formel (I), in welcher die Reste

$R^1$ bis $R^4$ für Wasserstoff, Chlor, Brom, Nitro, Cyano, Methyl, Methoxy, Ethoxy, Methylthio, Trifluormethyl, Trifluormethoxy oder Bis-(trifluormethyl)-amino stehen,

n für Null oder 1 steht,

$R^5$ für Alkyl, Alkoxyalkyl, Alkenyl oder Alkinyl, jeweils mit bis zu 10 Kohlenstoffatomen und - für den Fall, daß n für Null steht - auch für Cyanoalkyl oder Alkylthioalkyl, jeweils mit bis zu 10 Kohlenstoffatomen, für Cycloalkyl mit 3 bis 12 Kohlenstoffatomen, für gegebenenfalls halogensubstituiertes Benzyl oder Phenethyl oder für Phenyl steht, welches durch gegebenenfalls

substituierte Reste aus der Reihe $C_1$-$C_4$-Alkyl, $C_1$-$C_4$-Alkoxy oder $C_1$-$C_4$-Alkylthio substituiert sein kann, in welcher weiter

$R^6$ für gegebenenfalls halogen-substituierte Reste aus der Reihe Alkyl, Alkenyl, Alkinyl, Alkoxyalkyl, Alkylthioalkyl, Cyanoalkyl, jeweils mit bis zu lo Kohlenstoffatomen, für Cycloalkyl mit 3 bis 12 Kohlenstoffatomen, für gegebenenfalls halogen-substituiertes Benzyl, Phenethyl oder Naphthyl oder für Phenyl steht, welches gegebenenfalls durch Halogen, Cyano, Nitro oder durch gegebenenfalls halogen-substituierte Reste aus der Reihe $C_1$-$C_4$-Alkyl, $C_1$-$C_4$-Alkoxy oder $C_1$-$C_4$-Alkylthio substituiert ist,

in welcher weiter - für den Fall, daß n für Null steht - die Reste $R^5$ und $R^6$ zusammen mit dem Stickstoffatom, an das sie gebunden sind, einen gegebenenfalls durch 1 bis 3 Alkylgruppen mit jeweils 1 bis 5 Kohlenstoffatomen substituierten, gegebenenfalls teilweise ungesättigten und/oder benzannelierten Monocyclus oder Bicyclus mit bis zu 15 Kohlenstoffatomen bilden, oder worin die Reste $R^5$ und $R^6$ zusammen mit dem Stickstoffatom, an das sie gebunden sind, einen gegebenenfalls durch 1 bis 3 Alkylgruppen mit jeweils 1 bis 5 Kohlenstoffatomen, durch Phenyl, welches gegebenenfalls durch $C_1$-$C_4$-Alkyl, $C_1$-$C_4$-Alkoxy, Halogen, $C_1$-$C_2$-Halogenalkyl oder Nitro substituiert ist, durch Benzyl oder Phenylethyl substituierten, gesättigten und ein weiteres Stickstoffatom, Sauerstoffatom oder Schwefelatom enthaltenden Monocyclus mit bis zu 5 Kohlenstoffatomen bilden.

Die vorliegende Erfindung betrifft insbesondere Verbindungen der Formel (I), in welcher die Reste

$R^1$ bis $R^4$ für Wasserstoff, Chlor, Brom, Methyl und/oder Trifluormethyl stehen,

n für Null oder 1 steht,

$R^5$ für $C_1$-$C_6$-Alkyl, $C_1$-$C_4$-Alkoxy-ethyl, Allyl, Propargyl, 1-Methyl-propargyl oder 1,1-Dimethylpropargyl oder - für den Fall, daß n für Null steht - auch für Cyanomethyl, Cyclopentyl, Cyclohexyl, Benzyl oder Phenyl steht,

in welcher weiter

$R^6$ für gegebenenfalls durch Fluor oder Chlor substituiertes $C_1$-$C_6$-Alkyl, $C_1$-$C_4$-Alkoxy-ethyl, Allyl, Propargyl, 1-Methyl-propargyl, 1,1-Dimethylpropargyl, Cyanomethyl, Cyclopentyl, Cyclohexyl, Benzyl, Naphthyl oder Pnenyl steht, welches gegebenenfalls durch Methyl, Chlor, Cyano, Nitro oder Methoxy substituiert ist, wobei auch mehrfache und gemischte Substitution durch die genannten Reste möglich ist,
in welcher weiter - für den Fall, daß n für Null steht - die Reste $R^5$ und $R^6$ zusammen mit dem Stickstoffatom, an das sie gebunden sind, für Pyrrolidyl, Monoalkyl- oder Dialkyl-pyrrolidyl mit 1 bis 3 Kohlenstoffatomen je Alkylgruppe, Morpholinyl, Monoalkyl- oder Dialkylmorpholinyl mit 1 bis 3 Kohlenstoffatomen je Alkylgruppe, Piperidyl, Monoalkyl-, Dialkyl- oder Trialkylpiperidyl mit 1 bis 3 Kohlenstoffatomen je Alkylgruppe,

für Perhydroazepinyl (Hexamethylenimino-Rest), für den Heptamethylenimino-Rest, für den Dodekamethylenimino-Rest, für Indolinyl, für Monoalkyl-, Dialkyl- oder Trialkyl-indolinyl mit bis zu 3 Kohlenstoffatomen je Alkylgruppe, für Perhydroindolyl, Monoalkyl-, Dialkyl- oder Trialkyl-perhydroindolyl mit 1 bis 3 Kohlenstoffatomen je Alkylgruppe, 1,2,3,4-Tetrahydrochinolyl oder 1,2,3,4-Tetrahydro-iso-chinolyl, Monoalkyl-, Dialkyl-oder Trialkyl-1,2,3,4-tetrahydrochinolyl oder -iso-chinolyl mit 1 bis 3 Kohlenstoffatomen je Alkylgruppe, für Perhydrochinolyl oder Perhydro-iso-chinolyl, Monoalkyl-, Dialkyl- oder Trialkylperhydrochinolyl oder -perhydroisochinolyl mit 1 bis 3 Kohlenstoffatomen je Alkylgruppe, für Perhydrothiazolyl, für Perhydrooxazolyl, für Perhydrooxazinyl, für den Rest

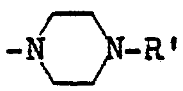

[worin R' für $C_1$-$C_4$-Alkyl, für gegebenenfalls durch $C_1$-$C_2$-Alkyl, $C_1$-$C_2$-Alkoxy, Fluor, Chlor, Brom, Trifluormethyl und/oder Nitro substituiertes Phenyl, für Benzyl oder Phenylethyl steht] oder für den Rest

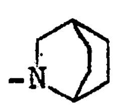

stehen.

Verwendet man bei der Herstellung der neuen Verbindungen der Formel (I) als Ausgangsstoffe beispielsweise 1-Hydroxy-benztriazol und Bromessigsäurepiperidid, so kann der Reaktionsablauf nach dem erfindungsgemäßen Herstellungsverfahren durch folgendes Formelschema wiedergegeben werden:

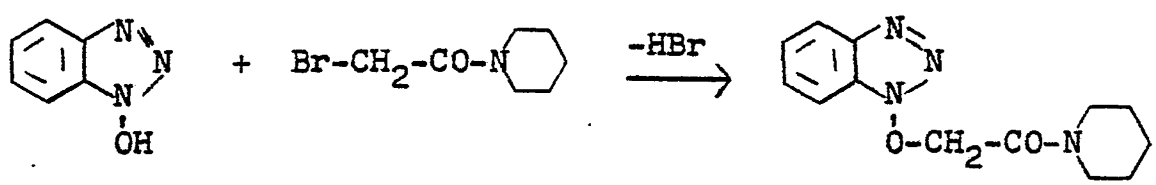


Die als Ausgangsstoffe zu verwendenden Hydroxybenztriazole sind durch die Formel (II) definiert. Vorzugsweise haben darin die Reste $R^1$, $R^2$, $R^3$ und $R^4$ die gleichen Bedeutungen, wie sie oben bei der vorzugsweisen Definition der entsprechenden Reste in Formel (I) genannt sind.

Als Beispiele seien genannt:
1-Hydroxy-benztriazol, 4-Chlor-, 5-Chlor-, 6-Chlor-, 5,6-Dichlor-, 6-Brom-, 6-Methyl-, 4-Chlor-6-methyl-, 5-Chlor-6-methyl-, 6-Trifluormethyl- und 5-Chlor-6-trifluormethyl-1-hydroxy-benztriazol.

Die Hydroxybenztriazole der Formel (II) sind bekannt und/oder können nach an sich bekannten Verfahren hergestellt werden (vgl. Houben-Weyl, Methoden der organischen Chemie, Band 1o/2, S. 236-24o, 4.Aufl. 1967, Thieme Verlag Stuttgart).

Die weiter als Ausgangsstoffe zu verwendenden Halogenessigsäureamide sind durch Formel (III) definiert. Vorzugsweise haben darin die Reste $R^5$ und $R^6$ die glei-

chen Bedeutungen, wie sie oben bei der vorzugsweisen Definition der entsprechenden Reste in Formel (I) genannt sind, und n steht für Null oder 1.

Als Beispiele seien genannt:
N-Methoxy-N-methyl-, N-Ethoxy-N-methyl-, N-n-Propoxy-N-methyl-, N-iso-Propoxy-N-methyl-, N-Ethoxy-N-ethyl-, N-n-Propoxy-N-ethyl-, N-iso-Propoxy-N-ethyl-, N-n-Propoxy-N-n-propyl-, N-iso-Propoxy-N-isopropyl-, N-iso-Propoxy-N-n-propyl-, N-Methoxy-N-ethyl-, N-Methoxy-N-n-propyl-, N-Methoxy-N-isopropyl-, N-Methoxy-N-n-butyl-, N-Methoxy-N-isobutyl-, N-Methoxy-N-sek.-butyl-, N-Methoxy-N-sek.-hexyl-, N-Ethoxy-N-n-propyl-, N-Ethoxy-N-isopropyl-, N-(2-Ethoxy-ethoxy)-N-methyl-, N-(2-Ethoxy-ethoxy)-N-ethyl-, N-(2-Ethoxy-ethoxy)-N-n-propyl-, N-(2-Ethoxy-ethoxy)-N-isopropyl-, N-(2-Ethoxy-ethoxy)-N-cyclohexyl-, N-Allyloxy-N-allyl-, N-Allyloxy-N-methyl-, N-Allyloxy-N-ethyl-, N-Allyloxy-N-n-propyl-, N-Allyloxy-N-isopropyl-, N-Allyloxy-N-n-butyl-, N-Allyloxy-N-iso-butyl-, N-Allyloxy-N-sek.-butyl-, N-Methoxy-N-cyclopentyl-, N-Methoxy-N-cyclohexyl-, N-Methoxy-N-(2-ethoxy-ethyl)-, N-Ethoxy-N-(2-ethoxy-ethyl)-, N-(2-Ethoxy-ethoxy)-N-(2-ethoxy-ethyl)- und N-(2-Ethoxy-ethoxy)-N-sek.-hexyl-chloressigsäureamid und -bromessigsäureamid, Chloressigsäure- und Bromessigsäuredimethylamid, -diethylamid, -di-n-propyl-amid, -di-iso-propylamid, -N-methyl-N-iso-propylamid, -N-methyl-N-iso-butylamid, -N-methyl-N-sek.-butylamid, -di-(2-ethyl-hexyl)-amid, -N-methyl-N-(2-cyano-ethyl)-amid, -di-(2-methoxy-ethyl)-amid, -di-allylamid, -N-methyl-N-propargylamid, -N-methyl-N-(1-methyl-propargyl)-amid, -dipropargylamid, -N-methyl-N-cyclopentylamid, -N-methyl-N-cyclohexyl-amid, -N-methyl-anilid, -N-methyl-N-(2-methyl-phenyl)-, -N-methyl-N-(3-methyl-phenyl)-, -N-methyl-N-(4-methyl-phenyl)-amid, -N-methyl-N-(2-chlorphenyl)-, -N-methyl-N-(3-chlorphenyl)-, -N-methyl-N-(4-chlor-phenyl)-amid, -N-methyl-N-(3-nitro-6-methyl-phenyl)-amid, -N-ethyl-anilid, -N-ethyl-N-(2-methyl-phenyl)-, -N-ethyl-N-(3-methyl-phenyl)-, -N-ethyl-N-(4-methyl-phenyl)-amid, -N-ethyl-N-(2-chlor-phenyl)-, -N-ethyl-N-(3-chlor-phenyl)-, -N-ethyl-N-(4-chlor-phenyl)-amid, -N-ethyl-N-(3-nitro-6-methyl-phenyl)-amid, -N-propyl-anilid, -N-propyl-N-(2-methyl-phenyl)-, -N-propyl-N-(3-methyl-phenyl)-, -N-propyl-N-(4-methyl-phenyl)-amid, -N-propyl-N-(2-chlor-phenyl)-, -N-propyl-N-(3-chlor-phenyl)-, -N-propyl-N-(4-chlor-phenyl)-amid, -N-iso-propyl-N-(2-methyl-phenyl)-, -N-iso-propyl-N-(3-methyl-phenyl)-, -N-iso-propyl-N-(4-methyl-phenyl)-amid, -N-iso-propyl-N-(3-nitro-6-methyl-phenyl)-amid, -N-butyl-anilid, -N-butyl-N-(2-methyl-phenyl)-, -N-butyl-N-(3-methyl-phenyl)-, -N-butyl-N-(4-methyl-phenyl)-amid, -N-butyl-N-(2-chlor-phenyl)-, -N-butyl-N-(3-chlor-phenyl)-, -N-butyl-N-(4-chlor-phenyl)-amid, -N-isobutyl-N-(2-methyl-phenyl)-, -N-iso-butyl-N-(3-methyl-phenyl)-, -N-iso-butyl-N-(4-methyl-phenyl)-amid, -N-iso-butyl-N-(3-nitro-6-methyl-phenyl)-amid, -N-methyl-N-naphth(1)ylamid, -N-methyl-N-naphth(2)ylamid, -N-ethyl-N-naphth(1)ylamid, -N-ethyl-N-naphth(2)ylamid, -N-n-propyl-N-naphth(2)yl-amid, -N-iso-propyl-N-naphth(2)ylamid, -N-n-butyl-N-naphth(2)ylamid, -N-iso-butyl-N-naphth(2)ylamid, -dibenzylamid, -N-methyl-N-benzylamid, -N-ethyl-N-benzylamid, -N-propyl-N-benzylamid, -N-butyl-N-benzylamid, -pyrrolidid, -2-methyl-pyrrolidid, -morpholid, -3,5-dimethyl-morpholid, -piperidid, -2-methyl-piperidid, -4-methyl-piperidid, -2,4-dimethyl-piperidid, -2,4,6-trimethyl-piperidid, -2-ethyl-piperidid, -4-ethyl-piperidid, -2,4-diethyl-piperidid, -2,4,6-triethyl-piperidid, 2-methyl-4-ethyl-piperidid, -2-ethyl-4-methyl-piperidid, -2-methyl-5-ethyl-piperidid, -2-ethyl-5-methyl-piperidid, -2-methyl-6-ethyl-piperidid, -1,2,3,4-tetrahydroindolid,
-perhydroindolid, -2-methyl-perhydroindolid, -2,2-dimethyl-perhydroindolid, -1,2,3,4-tetrahydrochinolid, -2-methyl-1,2,3,4-tetrahydrochinolid, -perhydrochinolid, -2-methyl-perhydrochinolid, -1,2,3,4-tetrahydro-isochinolid und -perhydroisochinolid.

Die Halogen-essigsäureamide der Formel (III) sind bekannt und/oder können nach an sich bekannten Verfahren hergestellt werden (vgl. BE-PS'en 800 250 und 844 501; DE-OS 2 o27 822; US-PS'en 2 921 075, 3 268 584, 3 647 876 und 4 196 142; J. Org.Chem. 24 (1959), 16o7-16o9).

Das Verfahren zur Herstellung der neuen 1-Benztriazolyloxyessigsäureamide wird vorzugsweise unter Verwendung geeigneter Lösungs- oder Verdünnungsmittel durchgeführt. Als solche kommen praktisch alle organischen Solventien infrage. Hierzu gehören insbesondere Alkohole, wie Methanol, Ethanol, n- und iso-Propanol, n-, iso-, sek.- und tert.-Butanol, Ether wie Diethylether, Dibutylether, Tetrahydrofuran und Dioxan, Ketone wie Aceton, Methylethylketon, Methylisopropylketon und Methylisobutylketon, Nitrile wie Acetonitril und Propionsäurenitril, sowie die hochpolaren Lösungsmittel Dimethylformamid, Dimethylsulfoxid, Sulfolan und Hexamethylphosphorsäuretriamid.

Als Säureakzeptoren können praktisch alle üblicherweise verwendbaren Säurebindemittel eingesetzt werden; hierzu gehören insbesondere Alkali- und Erdalkalihydroxide bzw. -oxide, z.B Calcium-hydroxid, Alkali- und Erdalkalicarbonate wie Natrium-, Kalium- und Calciumcarbonat, Alkalialkoholate wie Natrium-methylat, -ethylat und tert.-butylat, Kaliummethylat, -ethylat und -tert.-

butylat, ferner aliphatische, aromatische oder heterocyclische Amine wie Triethylamin, Dimethylanilin, Dimethylbenzylamin, Pyridin, Diazabicyclononen und Diazabicycloundecen.

Die Reaktionstemperatur kann innerhalb eines größeren Bereichs variiert werden. Im allgemeinen arbeitet man zwischen -5o und + 15o°C, vorzugsweise bei -2o bis +1oo°C. Das erfindungsgemäße Verfahren wird im allgemeinen bei Normaldruck durchgeführt.

Bei der Durchführung des erfindungsgemäßen Verfahrens setzt man auf 1 Mol 1-Hydroxy-benztriazol der Formel (II) 1,0 bis 1,5 Mol Halogen-essigsäureamid der Formel (III) ein. Die Umsetzung wird im allgemeinen in einem geeigneten Verdünnungsmittel durchgeführt und das Reaktionsgemisch wird bei der erforderlichen Temperatur mehrere Stunden gerührt.

Die Isolierung der Produkte erfolgt nach üblichen Methoden: Man destilliert gegebenenfalls einen Teil des Verdünnungsmittels unter vermindertem Druck ab und gießt den Rest der Reaktionsmischung in Wasser. Soweit die Produkte hierbei kristallin anfallen, werden sie durch Absaugen isoliert. Andernfalls werden die organischen Produkte mit einem mit Wasser nicht mischbaren Lösungsmittel, wie z.B. Toluol oder Methylenchlorid, extrahiert; nach Waschen und Trocknen wird dann von der organischen Phase das Lösungsmittel im Vakuum abdestilliert. Die zurückbleibenden Produkte werden durch ihren Schmelzpunkt bzw. ihren Brechungsindex charakterisiert.



BAD ORIGINAL

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.



BAD ORIGINAL

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen insbesondere neben einer sehr guten Wirkung gegen grasartige Unkräuter (eingeschlossen Cyperus) auch eine gute herbizide Wirkung bei breitblättrigen Unkräutern. Ein selektiver Einsatz der erfindungsgemäßen Wirkstoffe ist in verschiedenen Kulturen möglich, z.B. in Rüben, Baumwolle, Mais, Reis, Weizen und anderen Getreidearten.

Die Wirkstoffe können in die üblichen Formulierungen übergeführt werden, wie Lösungen, Emulsionen, Spritzpulver,



BAD ORIGINAL

Suspensionen, Pulver, Stäubemittel, Pasten, lösliche Pulver, Granulate, Suspensions-Emulsions-Konzentrate, wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone wie Aceton, Methyläthylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hoch-



BAD ORIGINAL

disperse Kieselsäure, Aluminiumoxid und Silicate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.



BAD ORIGINAL

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen oder Stäuben.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die



BAD ORIGINAL

Aufwandmengen zwischen 0,1 und 10 kg Wirkstoff pro ha, vorzugsweise zwischen 0,1 und 5 kg/ha.

Die nachfolgenden Beispiele dienen zur weiteren Erläuterung der Erfindung.



BAD ORIGINAL

## Herstellungsbeispiele

## Allgemeine Herstellungsvorschrift

o,1 Mol 1-Hydroxybenztriazol der Formel (II) und o,1 Mol Halogen-essigsäureamid der Formel (III) werden mit o,12 Mol Kaliumcarbonat in 15o ml Acetonitril vermischt und dieses Reaktionsgemisch wird bis zum Ende der Umsetzung unter Rückfluß zum Sieden erhitzt und gerührt. Das Lösungsmittel wird dann unter vermindertem Druck weitgehend abdestilliert und der Rückstand in Toluol aufgenommen. Diese Lösung wird mit Wasser, verdünnter Natronlauge und wieder mit Wasser gewaschen, über Natriumsulfat getrocknet und mit Aktivkohle/Tonsil geklärt. Nach Filtrieren wird vom Filtrat das Lösungsmittel unter vermindertem Druck sorgfältig abdestilliert. Zur Reinigung wird mit Petrolether verrieben, abgesaugt und getrocknet oder - falls keine Kristallisation erfolgt - bei 1oo°C "andestilliert", d.h. längere Zeit am Dampfstrahlvakuum gehalten.
Nach dieser Herstellungsvorschrift wurden beispielsweise die in der nachstehenden Tabelle I aufgeführten Verbindungen der Formel I (mit n = Null) erhalten:

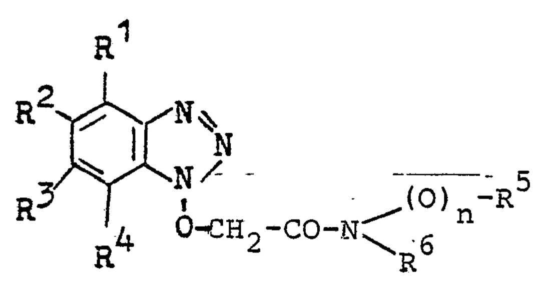

(I)



BAD ORIGINAL

Tabelle I: Verbindungen der Formel I ( mit n = 0)

| Verbindung Nr. | $R^1$ | $R^2$ | $R^3$ | $R^4$ | $R^5$ | $R^6$ | Schmelzpunkt (°C) bzw. Brechungsindex $n_D^{20}$: |
|---|---|---|---|---|---|---|---|
| 1 | H | H | H | H | $-CH_2-CH=CH_2$ | $CH_2-CH_2=CH_2$ | 160 |
| 2 | H | H | H | H | $-CH_3$ | $-CH_2-$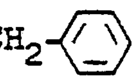 | 1,5875 |
| 3 | H | H | H | H | $-C_2H_5$ | 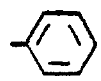 | 1,5821 |
| 4 | H | H | H | H | $-CH_3$ | $-CH_2CN$ | zähes Öl |
| 5 | H | H | H | H | $-CH_3$ | $-C_6H_{11}$ | |
| 6 | H | H | H | H | 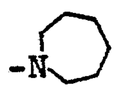 | | 96 |
| 7 | H | H | H | H | 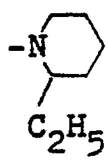 | | 1,5903 |
| 8 | H | H | H | H | 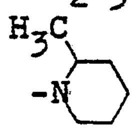 | | 136 |

Tabelle I (Fortsetzung)

| Verbindung Nr. | $R^1$ | $R^2$ | $R^3$ | $R^4$ | $R^5$ | $R^6$ | Schmelzpunkt (°C) bzw. Brechungsindex $n_D^{20}$: |
|---|---|---|---|---|---|---|---|
| 9 | H | H | Cl | H | 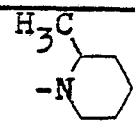 | | 52-6o |
| 10 | H | Cl | H | H | $-CH_3$ | $-C_6H_5$ | 126-29 |
| 11 | Cl | H | H | H | $-CH_3$ | $-C_6H_5$ | 122-24 |
| 12 | H | Cl | Cl | H | $-CH_3$ | $-C_6H_5$ | 13o-141 |
| 13 | H | Cl | Cl | H | 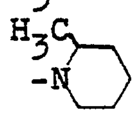 | | 12o-129 |
| 14 | Cl | H | H | H | 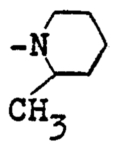 | | 12o-124 |
| 15 | H | Cl | H | H | 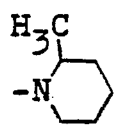 | | 1o6 |
| 16 | Cl | H | H | H | $-CH_3$ | $-C_6H_{11}$ | 97 |
| 17 | H | Cl | Cl | H | $-CH_3$ | $-C_6H_{11}$ | 165 |
| 18 | H | Cl | H | H | $-CH_3$ | $-C_6H_{11}$ | 145 |
| 19 | H | H | $CF_3$ | H | 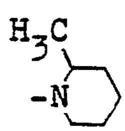 | | zähes Öl |
| 20 | H | H | $CF_3$ | H | $-CH_3$ | $-C_6H_5$ | 1o8-118 |

Tabelle I (Fortsetzung)

| Verbindung Nr. | $R^1$ | $R^2$ | $R^3$ | $R^4$ | $R^5$ | $R^6$ | Schmelzpunkt (°C) bzw. Brechungsindex $n_D^{20}$: |
|---|---|---|---|---|---|---|---|
| 21 | H | Cl | $CH_3$ | H | $-CH_3$ | $-C_6H_5$ | 1o6-112 |
| 22 | H | Cl | $CH_3$ | H | 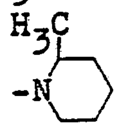 | | 1o4 |
| 23 | H | Cl | $CH_3$ | H | $-CH_3$ | $-C_6H_{11}$ | 153 |
| 24 | H | H | $CH_3$ | H | $-CH_3$ | $-C_6H_5$ | 111 |
| 25 | H | H | $CH_3$ | H | 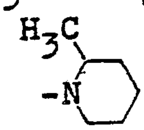 | | zähes Öl |
| 26 | H | H | $CH_3$ | H | $-CH_3$ | $-C_6H_{11}$ | zähes Öl |
| 27 | H | Cl | H | H | 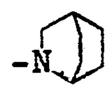 | | 125 |
| 28 | H | H | Cl | H | 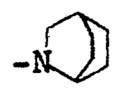 | | 96 |
| 29 | H | H | $CH_3$ | H | 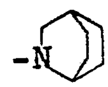 | | 90-92 |
| 3o | H | H | $CF_3$ | H | 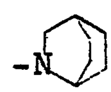 | | 88-91 |
| 31 | H | Cl | $CH_3$ | H | 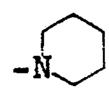 | | 13o-132 |
| 32 | $CH_3$ | Cl | H | H | $-CH_3$ | $-C_6H_5$ | 121-124 |
| 33 | H | H | Br | H | $-CH_3$ | $-C_6H_5$ | 99 |
| 34 | Cl | H | Cl | Cl | $-CH_3$ | $-C_6H_5$ | 2o8 |

Tabelle I (Fortsetzung)



| Verbindung Nr. | $R^1$ | $R^2$ | $R^3$ | $R^4$ | $R^5$ | $R^6$ | Schmelzpunkt (°C) bzw. Brechungsindex $n_D^{20}$: |
|---|---|---|---|---|---|---|---|
| 35 | H | H | H | H | 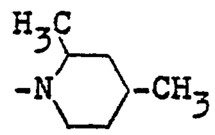 | | 1,5461/23° |
| 36 | H | H | H | H | 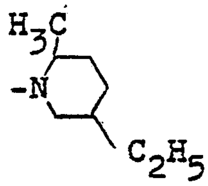 | | 133 |
| 37 | H | H | H | H | $-C_2H_5$ | $-C_3H_7$-iso | 65 |
| 38 | H | H | H | H | 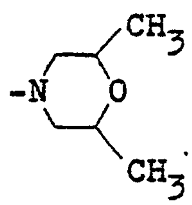 | | 1,5582/23° |

Ebenso erhält man die in der nachstehenden Tabelle II aufgeführten Verbindungen der Formel I (mit n = 1):

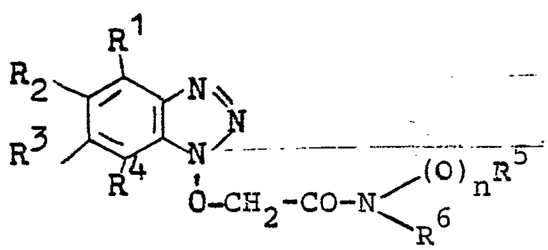 (I)

**Tabelle II:** Verbindungen der Formel I (mit n = 1)

| Verbindung Nr. | $R^1$ | $R^2$ | $R^3$ | $R^4$ | $R^5$ | $R^6$ | Schmelzpunkt (°C) bzw. Brechungsindex $n_D^{22}$: |
|---|---|---|---|---|---|---|---|
| 39 | H | H | Cl | H | $CH_2-CH_2-OC_2H_5$ | $C_3H_7$-iso | 1,4945 |
| 40 | H | H | Cl | H | $C_3H_7$-iso | $C_3H_7$-iso | 83 |
| 41 | H | Cl | H | H | $CH_2-CH_2-OC_2H_5$ | $C_3H_7$-iso | 1,5050 |
| 42 | H | Cl | H | H | $CH_3$ | $CH_3$ | 79 |
| 43 | H | Cl | $CH_3$ | H | $CH_2-CH_2-OC_2H_5$ | $C_3H_7$-iso | 1,5256 |
| 44 | H | Cl | $CH_3$ | H | $C_3H_7$-n | $C_3H_7$-n | 1,5320 |
| 45 | H | H | $CH_3$ | H | $CH_2-CH_2-OC_2H_5$ | $C_3H_7$-iso | 1,5126 |

Analog der allgemeinen Herstellungsvorschrift wurden auch folgende Verbindungen der Formel (I) hergestellt:

Tabelle III

| Verbindung Nr. | Schmelzpunkt (Fp.) bzw. Brechungsindex ($n_D$) |
|---|---|
| 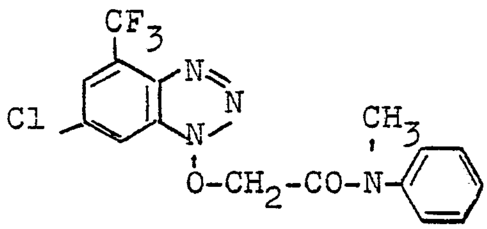 (46) | Fp. 130°C |
| 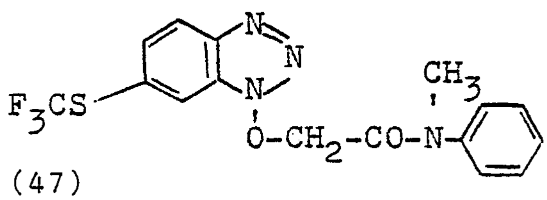 (47) | Fp. 112°C |
| 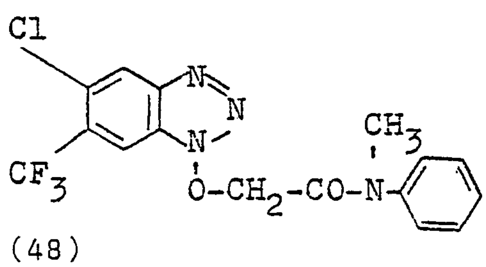 (48) | Fp. 125°C |
| 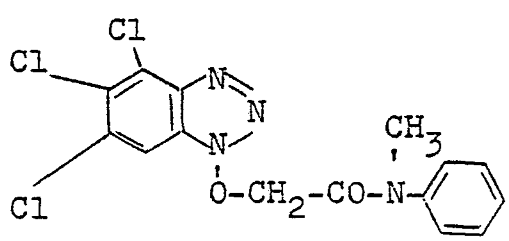 (49) | Fp. 119°C |

Tabelle III (Fortsetzung)

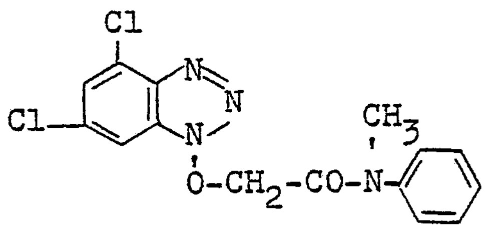

Fp. 127°C

(5o)

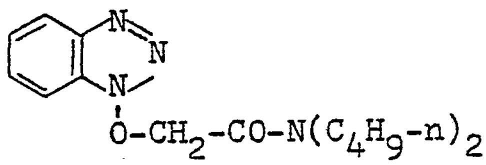

$n_D^{20}$: 1,5240

(51)

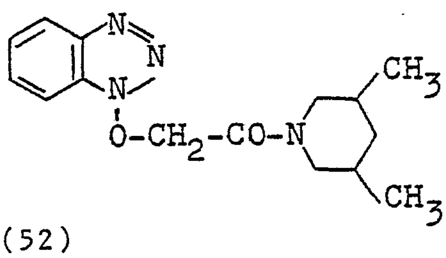

Fp. 98°C

(52)

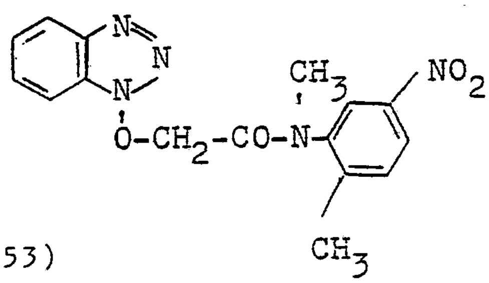

Fp. 173°C

(53)

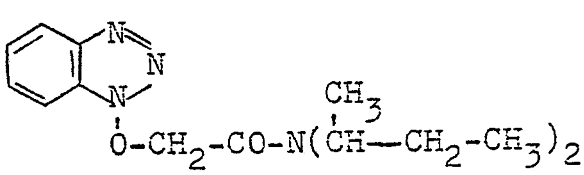

Fp. 94-95°C

(54)

Tabelle III (Fortsetzung)

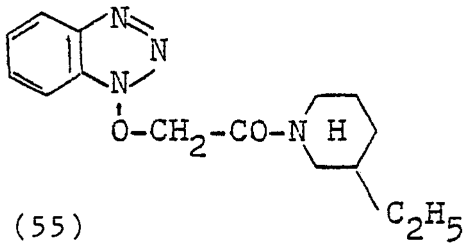

(55)

$n_D^{20}$: 1,5160

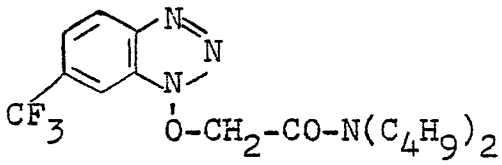

(56)

$n_D^{20}$: 1,4950

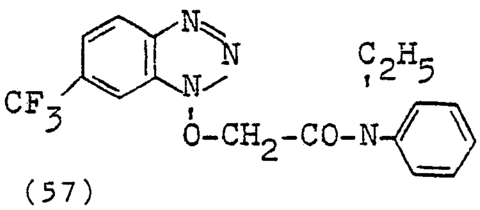

(57)

Fp. 98°C

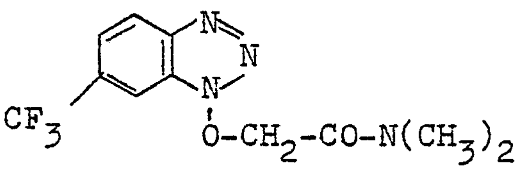

(58)

Fp. 98°C

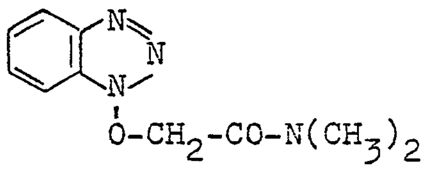

(59)

$n_D^{20}$: 1,5684

0048850



Tabelle III (Fortsetzung)

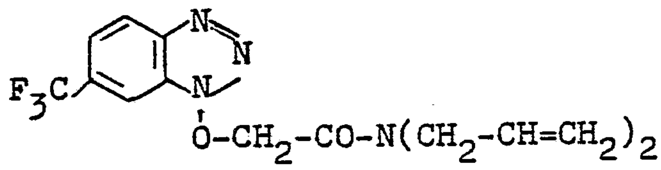

$n_D^{20}$: 1,5152

(6o)

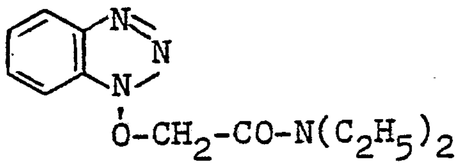

Fp. 55°C

(61)

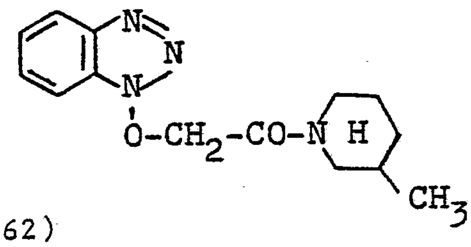

Fp. 83°C

(62)

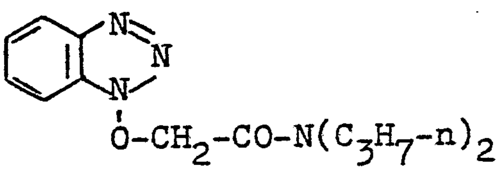

Fp. 83°C

(63)

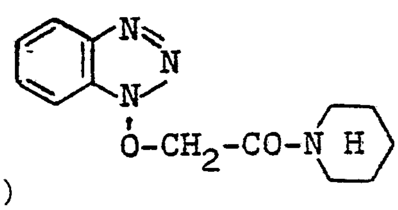

Fp.88°C

(64)

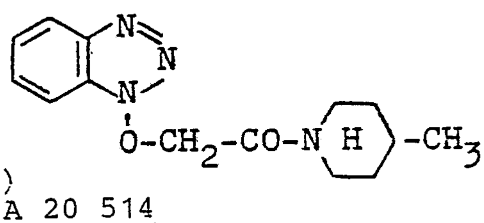

Fp. 78°C

(65)

Tabelle III (Fortsetzung)

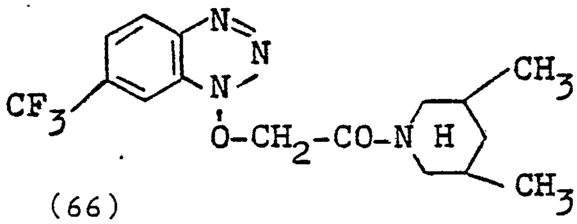

(66)

$n_D^{20}$: 1,5121

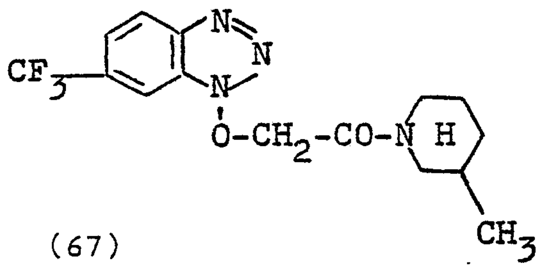

(67)

$n_D^{20}$:1,5168

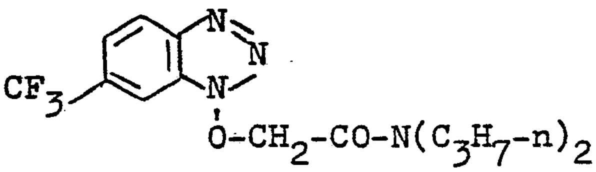

(68)

$n_D^{20}$:1,4985

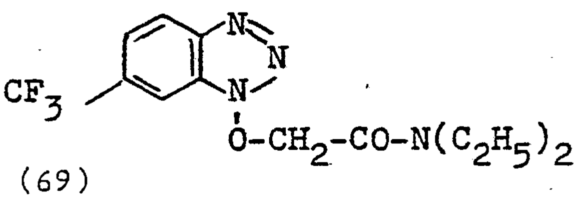

(69)

$n_D^{20}$:1.5049

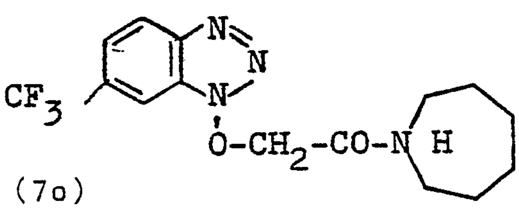

(70)

Fp. 54°C

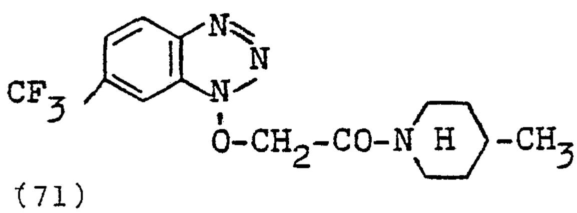

(71)

Fp. 91°C

## Verwendungsbeispiele

### Beispiel A

Pre-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test zeigen z.B. die folgenden Verbindungen der Herstellungsbeispiele eine ausgezeichnete Wirkung:

Nr. 6,10, 11, 12, 13, 14, 16, 19, 20, 21, 28, 32, 35,48, 53.

Als Vergleichsmittel diente das bekannte 2,4-Dichlorphenoxyessigsäureamid (2,4-D-amid).

Patentansprüche

1) Benztriazolyloxy-essigsäureamide der allgemeinen Formel (I)

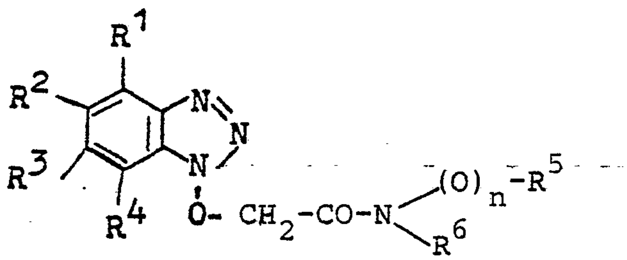

(I)

in welcher

$R^1$, $R^2$, $R^3$ und $R^4$, welche gleich oder verschieden sein können, einzeln für Wasserstoff, Cyano, Nitro, Halogen, Amino oder gegebenenfalls halogensubstituiertes (Di)Alkylamino, Carbamoyl, Monoalkylamino- oder Dialkylamino-carbonyl, Aminosulfonyl, Monoalkylamino- oder Dialkylaminosulfonyl, für gegebenenfalls durch Halogen, Cyano, Alkoxy oder Alkylthio substituiertes Alkyl, oder für gegebenenfalls halogen-substituiertes Alkoxy, Alkylthio oder Alkylsulfonyl stehen, oder in welcher zwei benachbarte Reste $R^1$ und $R^2$, $R^2$ und $R^3$ oder $R^3$ und $R^4$ zusammen für Alkylen oder Benzo stehen,

n für Null oder 1 steht und

$R^5$ und $R^6$, welche gleich oder verschieden sein können, einzeln für gegebenenfalls substituierte Reste aus der Reihe Alkyl, Alkenyl, Alkinyl, Cycloalk(en)yl, Aralkyl oder Aryl stehen

oder - für den Fall, daß n für Null steht - zusammen mit dem Stickstoffatom an das sie gebunden sind, einen gegebenenfalls substituierten, gegebenenfalls teilweise ungesättigten und gegebenenfalls benzannelierten Mono- oder Bicyclus bilden, der gegebenenfalls weitere Heteroatome enthält.

2) 1-Benztriazolyloxy-essigsäureamide der allgemeinen Formel (I) gemäß Anspruch 1, dadurch gekennzeichnet, daß

$R^1$, $R^2$, $R^3$ und $R^4$, welche gleich oder verschieden sein können, für Wasserstoff, Chlor, Brom, Nitro, Cyano, Methyl, Methoxy, Ethoxy, Methylthio, Trifluormethyl, Trifluormethoxy oder Bis-(trifluormethyl)-amino stehen,

n für Null oder 1 steht,

$R^5$ für Alkyl, Alkoxyalkyl, Alkenyl oder Alkinyl, jeweils mit bis zu 10 Kohlenstoffatomen und - für den Fall, daß n für Null steht - auch für Cyanoalkyl oder Alkylthioalkyl, jeweils mit bis zu 10 Kohlenstoffatomen, für Cycloalkyl mit 3 bis 12 Kohlenstoffatomen, für gegebenenfalls halogensubstituiertes Benzyl oder Phenethyl oder für Phenyl steht, welches durch gegebenenfalls substituierte Reste aus der Reihe $C_1$-$C_4$-Alkyl, $C_1$-$C_4$-Alkoxy oder $C_1$-$C_4$-Alkylthio substituiert sein kann,

$R^6$ für gegebenenfalls halogen-substituierte Reste aus der Reihe Alkyl, Alkenyl, Alkinyl, Alkoxyalkyl, Alkylthioalkyl, Cyanoalkyl, jeweils mit bis zu 10 Kohlenstoffatomen, für Cycloalkyl mit 3 bis 12 Kohlenstoffatomen, für gegebenenfalls halogen-substituiertes Benzyl, Phenethyl oder Naphthyl oder für Phenyl steht, welches gegebenenfalls durch Halogen, Cyano, Nitro oder durch gegebenenfalls halogen-substituierte Reste aus der Reihe $C_1$-$C_4$-Alkyl, $C_1$-$C_4$-Alkoxy oder $C_1$-$C_4$-Alkylthio substituiert ist, oder daß - für den Fall, daß n für Null steht - die Reste $R^5$ und $R^6$ zusammen mit dem Stickstoffatom, an das sie gebunden sind, einen gegebenenfalls durch 1 bis 3 Alkylgruppen mit jeweils 1 bis 5 Kohlenstoffatomen substituierten, gegebenenfalls teilweise ungesättigten und/oder benzannelierten Monocyclus oder Bicyclus mit bis zu 15 Kohlenstoffatomen bilden, oder daß die Reste $R^5$ und $R^6$ zusammen mit dem Stickstoffatom, an das sie gebunden sind, einen gegebenenfalls durch 1 bis 3 Alkylgruppen mit jeweils 1 bis 5 Kohlenstoffatomen, durch Phenyl, welches gegebenenfalls durch $C_1$-$C_4$-Alkyl, $C_1$-$C_4$-Alkoxy, Halogen, $C_1$-$C_2$-Halogenalkyl oder Nitro substituiert ist, durch Benzyl oder Phenylethyl substituierten, gesättigten und ein weiteres Stickstoffatom, Sauerstoffatom oder Schwefelatom enthaltenden Monocyclus mit bis zu 5 Kohlenstoffatomen bilden.

3) 1-Benztriazolyloxy-essigsäureamide der allgemeinen Formel (I) gemäß Anspruch 1, dadurch gekennzeichnet, daß

$R^1$ bis $R^4$ für Wasserstoff, Chlor, Brom, Methyl und/ oder Trifluormethyl stehen,

n für Null oder 1 steht,

$R^5$ für $C_1$-$C_6$-Alkyl, $C_1$-$C_4$-Alkoxy-ethyl, Allyl, Propargyl, 1-Methyl-propargyl oder 1,1-Dimethylpropargyl oder - für den Fall, daß n für Null steht - auch für Cyanomethyl, Cyclopentyl, Cyclohexyl, Benzyl oder Phenyl steht,

$R^6$ für gegebenenfalls durch Fluor oder Chlor substituiertes $C_1$-$C_6$-Alkyl, $C_1$-$C_4$-Alkoxy-ethyl, Allyl, Propargyl, 1-Methyl-propargyl, 1,1-Dimethylpropargyl, Cyanomethyl, Cyclopentyl, Cyclohexyl, Benzyl, Naphthyl oder Phenyl steht, welches gegebenenfalls durch Methyl, Chlor, Cyano, Nitro oder Methoxy substituiert ist, wobei auch mehrfache und gemischte Substitution durch die genannten Reste möglich ist, oder daß - für den Fall, daß n für Null steht - die Reste $R^5$ und $R^6$ zusammen mit dem Stickstoffatom, an das sie gebunden sind, für Pyrrolidyl, Monoalkyl- oder Dialkyl-pyrrolidyl mit 1 bis 3 Kohlenstoffatomen je Alkylgruppe, Morpholinyl, Monoalkyl- oder Dialkylmorpholinyl mit 1 bis 3 Kohlenstoffatomen je Alkylgruppe, Piperidyl, Monoalkyl- , Dialkyl- oder Trialkylpiperidyl mit 1 bis 3 Kohlenstoffatomen je Alkylgruppe,

für Perhydroazepinyl (Hexamethylenimino-Rest), für den Heptamethylenimino-Rest, für den Dodecamethylenimino-Rest, für Indolinyl, für Monoalkyl-, Dialkyl- oder Trialkyl-indolinyl mit bis zu 3 Kohlenstoffatomen je Alkylgruppe, für Perhydroindolyl, Monoalkyl-, Dialkyl- oder Trialkyl-perhydroindolyl mit 1 bis 3 Kohlenstoffatomen je Alkylgruppe, 1,2,3,4-Tetrahydrochinolyl oder 1,2,3,4-Tetrahydro-iso-chinolyl, Monoalkyl-, Dialkyl- oder Trialkyl-1,2,3,4-tetrahydrochinolyl oder -iso-chinolyl mit 1 bis 3 Kohlenstoffatomen je Alkylgruppe, für Perhydrochinolyl oder Perhydro-iso-chinolyl, Monoalkyl-, Dialkyl- oder Trialkylperhydrochinolyl oder -perhydroisochinolyl mit 1 bis 3 Kohlenstoffatomen je Alkylgruppe, für Perhydrothiazolyl, für Perhydrooxazolyl, für Perhydrooxazinyl, für den Rest

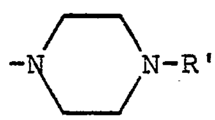

(worin R' für $C_1$-$C_4$-Alkyl, für gegebenenfalls durch $C_1$-$C_2$-Alkyl, $C_1$-$C_2$-Alkoxy, Fluor, Chlor, Brom, Trifluormethyl und/oder Nitro substituiertes Phenyl, für Benzyl oder Phenylethyl steht) oder für den Rest

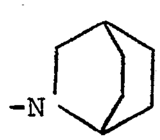

stehen.

4) Verfahren zur Herstellung von 1-Benztriazolyloxyessigsäureamiden der Formel (I) gemäß Anspruch 1, dadurch gekennzeichnet, daß man 1-Hydroxybenztriazole der allgemeinen Formel (II)

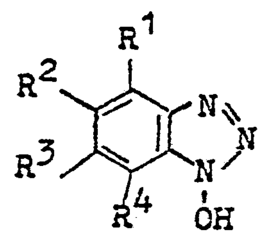

(II)

in welcher

$R^1$, $R^2$, $R^3$ und $R^4$ die oben angegebene Bedeutungen haben,

mit Halogen-essigsäureamiden der allgemeinen Formel (III)

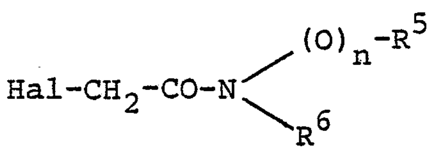

(III)

in welcher

n, $R^5$ und $R^6$ die oben angegebene Bedeutung haben und

Hal für Chlor oder Brom steht,

gegebenenfalls in Gegenwart eines Säureakzeptors und gegebenenfalls unter Verwendung eines Verdünnungsmittels umsetzt.

5) Herbizide Mittel, gekennzeichnet durch einen Gehalt an 1-Benztriazolyloxy-essigsäureamiden der Formel (I) gemäß Anspruch 1.

6) Verfahren zur Bekämpfung von unerwünschtem Pflanzenwachstum, dadurch gekennzeichnet, daß man 1-Benztriazolyloxy-essigsäureamide der Formel (I) gemäß Anspruch 1 auf die unerwünschten Pflanzen oder ihren Lebensraum einwirken läßt.

7) Verwendung von 1-Benztriazolyloxy-essigsäureamiden der Formel (I) gemäß Anspruch 1 zur Bekämpfung von Pflanzenwachstum.

8) Verfahren zur Herstellung von herbiziden Mitteln, dadurch gekennzeichnet, daß man 1-Benztriazolyloxy-essigsäureamide der Formel (I) gemäß Anspruch 1 mit Streckmitteln und/oder oberflächenaktiven Mitteln vermischt.

Europäisches Patentamt

# EUROPÄISCHER RECHERCHENBERICHT

0048850

Nummer der Anmeldung

EP 81 10 7095

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | US - A - 4 174 285 (BRAID) | 1 |
| PA | EP - A - 0 029 160 (BAYER)<br>* Ansprüche *<br>-- | 1-8 |
| PA | EP - A - 0 029 183 (BAYER)<br>* Ansprüche *<br>---- | 1-8 |

KLASSIFIKATION DER ANMELDUNG (Int. Cl.³)

C 07 D 249/18
249/22
249/16
401/12
403/12
413/12
453/06
A 01 N 43/64
43/72

RECHERCHIERTE SACHGEBIETE (Int. Cl.³)

C 07 D 249/12
453/06
403/12
413/12
401/12

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 22-12-1981 | CREMERS |

EPA form 1503.1 06.78